# EUROPÄISCHE PATENTSCHRIFT

(11) **EP 1 624 808 B1**
(45) Veröffentlichungstag und Bekanntmachung des Hinweises auf die Patenterteilung: **10.12.2008**
(21) Anmeldenummer: 04732912.3
(22) Anmeldetag: 14.05.2004
(51) Int. Cl.: A61B 17/00, B05C 17/005

(54) **AUSTRAGANORDNUNG FUER ZWEI KOMPONENTEN MIT AUSTRAGKARTUSCHE UND MISCHER**
TWO-COMPONENT DISPENSING SYSTEM COMPRISING A DISPENSER CARTRIDGE AND A MIXER
DISPOSITIF DE DECHARGE POUR DEUX COMPOSANTS AVEC CARTOUCHE DE DECHARGE ET MELANGEUR

(30) Priorität: 19.05.2003 CH 8842003
(43) Veröffentlichungstag der Anmeldung: 15.02.2006
(73) Patentinhaber: Medmix Systems AG, 6343 Rotkreuz (CH)
(72) Erfinder: KELLER, Wilhelm A., CH-6402 Merlischachen (CH)
(74) Vertreter: AMMANN PATENTANWÄLTE AG BERN
(86) Internationale Anmeldenummer: PCT/CH2004/000295
(87) Internationale Veröffentlichungsnummer: WO 2004/100798

(56) Entgegenhaltungen:
- WO-A-01/24869
- WO-A-01/51218
- WO-A-01/74253
- WO-A-97/28834
- WO-A-99/32173
- WO-A-03/039375
- CH-A- 350 241
- GB-A- 1 531 416

## Beschreibung

Bei den meisten Austraganordnungen wird der Mischer an der Kartusche befestigt und diese Anordnung in dieser Form benutzt. Oft wird am Mischer ein Zusatz angebracht, um die Form der vermischten Komponenten zu beeinflussen und um gezielt bei speziellen Anordnungen das Material an Ort und Stelle zu applizieren. Es gibt jedoch eine Anzahl von Anwendungen, insbesondere in der Medizin, bei denen eine solche einfache Anordnung Austragkartusche-Mischer-Zusatzvorrichtung nicht genügt.

In der Medizin ist man dazu übergegangen, bestimmte Verbindungen nicht mehr zu nähen sondern zu kleben, wozu ein Zweikomponentenkleber verwendet wird. Beispielsweise für die Verwendung von Austraganordnungen bei Operationen, bei denen ein Endoskop verwendet wird ist es notwendig, ein langes Transferteil zwischen Kartusche und Mischer zu verwenden, wobei dies insbesondere bei der Laparoskopie zutrifft. Bei der Laparoskopie werden relativ lange Rohre in die Bauchhöhle hineingebracht, so dass der Abstand zwischen Kartusche und Austragspitze recht gross werden kann.

Aus der WO-A2-03/039375 ist eine Laparoskopie-Sprühvorrichtung gemäss Oberbegriff von Patentanspruch 1 bekannt, die ein rohrartiges Transferteil aufweist, an dem eingangseitig eine Austragvorrichtung und ausgangseitig eine Sprühvorrichtung ankoppelbar ist, wobei die Sprühvorrichtung eine Mischkammer mit einem flexiblen Mischorgan aufweist. Das Transferteilrohr weist zwei Längsbohrungen auf. Die Herstellung von Rohren mit dünnen Längsbohrungen kann problematisch werden, falls längere Rohre benötigt werden.

Aus der GB-A-1 531 416 ist eine Kurette bekannt, die ein Rohr aufweist, in welchem aussen zwei Nuten angeordnet sind, die von einem Mantel umgeben sind, um Durchlässe für Luft zu bilden und aus der CH-A-350 241 ist eine Abgabevorrichtung bekannt, die zum Austragen von Zahnpasta in verschiedenen Farben ein Innenrohr mit Nuten aufweist, das ummantelt ist.

Bei der Verwendung von Austraganordnungen für die Medizin ist es wichtig, dass einerseits das Mischungsverhältnis der Komponenten von anfang an genau eingehalten wird und dass andererseits keine Luft ausgetragen wird.

Es ist davon ausgehend Aufgabe der vorliegenden Erfindung eine Austraganordnung anzugeben, bei welcher eine Austragkartusche oder ein Austraggerät mittels einem Transferteil mit einem relativ weit entfernten Mischer oder einem anderen Zubehörteil, z. B. Sprühkopf, verbindbar und kostengünstig herstellbar ist und die eine genaue und luftfreie Abgabe des Gemisches erlaubt. Eine solche Anordnung ist in Patentanspruch 1 beschrieben.

Die Erfindung wird im Folgenden anhand von Zeichnungen eines Ausführungsbeispiels näher erläutert.
- Figur 1: zeigt in einem Längsschnitt eine Austraganordnung gemäss Erfindung mit einer aufgesetzten Ausgleichvorrichtung,
- Figur 2: zeigt eine Ausschnittvergrösserung von Figur 1,
- Figur 3: zeigt einen Schnitt gemäss der Linie III - III in Figur 2,
- Figur 4: zeigt eine erfindungsgemässe Austraganordnung mit aufgesetztem Mischer, und
- Figur 5: zeigt eine Ausschnittvergrösserung von Figur 3
- Figur 6: zeigt einen Schnitt gemäss der Linie VI - VI in Figur 5.

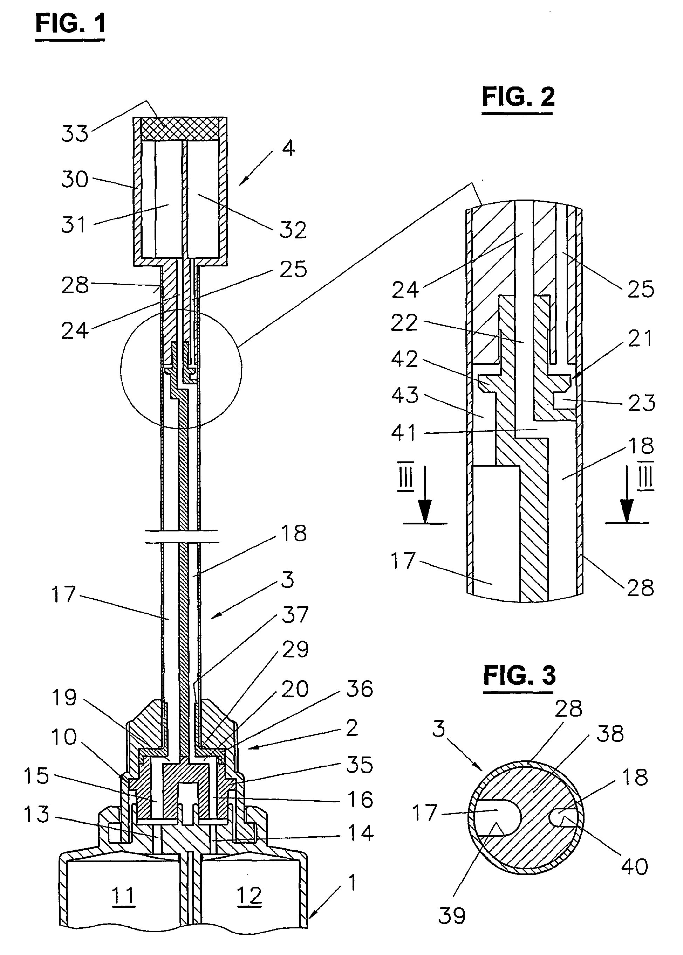

Figur 1 zeigt eine Doppelkartusche 1 mit Überwurfring 2 zur Befestigung eines Transferteiles 3 und eine darauf aufgesteckte Ausgleichvorrichtung 4. Der Überwurfring und die Kartusche besitzen an sich bekannte Bayonett-Befestigungsmittel. Es sind auch andere Befestigungsmittel, wie z.B. Schnappring, möglich. Die Ausgleichvorrichtung dient dazu, einerseits den Füllstand der Komponenten anzugleichen, damit das Mischungsverhältnis der Komponenten vom Anfang des Austrags bzw. Mischvorgangs genau eingehalten werden kann und andererseits, die Vorratszylinder der Doppelkartusche vollständig zu entlüften, damit keine Luft darin eingeschlossen wird. Diese beiden Bedingungen sind in der Medizinaltechnik besonders wichtig.

Bei der Verwendung für die Endoskopie, bzw. Laparoskopie, dürfen das Transferteil und der Mischer einen bestimmten, geringen Durchmesser nicht überschreiten und müssen auch ohne Absatz ausgebildet sein. Da jedoch aus wirtschaftlichen Überlegungen auf dem Markt erhältliche Kartuschen verwendet werden, die auch für andere Anwendungen gedacht sind und deren nebeneinanderliegenden oder konzentrischen Auslässe einen Gesamtdurchmesser ergeben, der wesentlich grösser ist als der gestattete Durchmesser des Transferteils, ist es erforderlich, zwischen der Kartusche und dem Transferteil einen Adapter anzuordnen, um den Übergang des Materials zwischen den Kartuschenauslässen und den Transferteil-Kanälen zu ermöglichen. Es ist zweckmässig, zwischen dem Transferteil und dem Mischer oder der Ausgleichvorrichtung ebenfalls einen Adapter vorzusehen.

Jeder Vorratszylinder 11 und 12 der Doppelkartusche besitzt einen Auslass 13 und 14, der je in einen Kanal 15 und 16 im Eingangs-Adapter 10 mündet. Im Ausführungsbeispiel sind die Volumina, bzw. die Querschnittsflächen und die Auslässe der Vorratszylinder voneinander verschieden, wobei das Verhältnis von 1:1 bis 1:10 je nach Verwendungszweck und Materialien variieren kann.

Der Eingangsadapter 10 ist aus fertigungstechnischen Gründen zweiteilig hergestellt und enthält den Adapterkörper 35 mit den Kanälen 15 und 16 sowie, ausgangsseitig, ein Flanschteil 36, auf dessen Rohrstück 37 das Mantelrohr 28 des Transferteils 3 aufgesetzt ist.

Das Transferteil weist eine zylinderförmige Stange 38 auf, um die ein Mantelrohr 28 angeordnet ist. Die Stange 38 weist zwei zueinander gegenüberliegend angeordnete Nuten 39 und 40, die mit dem Mantelrohr zwei Kanäle 17 und 18 ergeben, siehe Figur 3. Dabei muss Sorge getragen werden, dass die beiden Nuten, bzw. Kanäle, abgedichtet voneinander getrennt sind, damit kein Material von einem Kanal zum anderen Kanal gelangen kann. Die Dichtung kann beispielsweise durch Aufpressen des Mantels um die Stange hergestellt werden, doch sind andere Dichtungsmöglichkeiten wie Dichtwulst oder Kleben möglich.

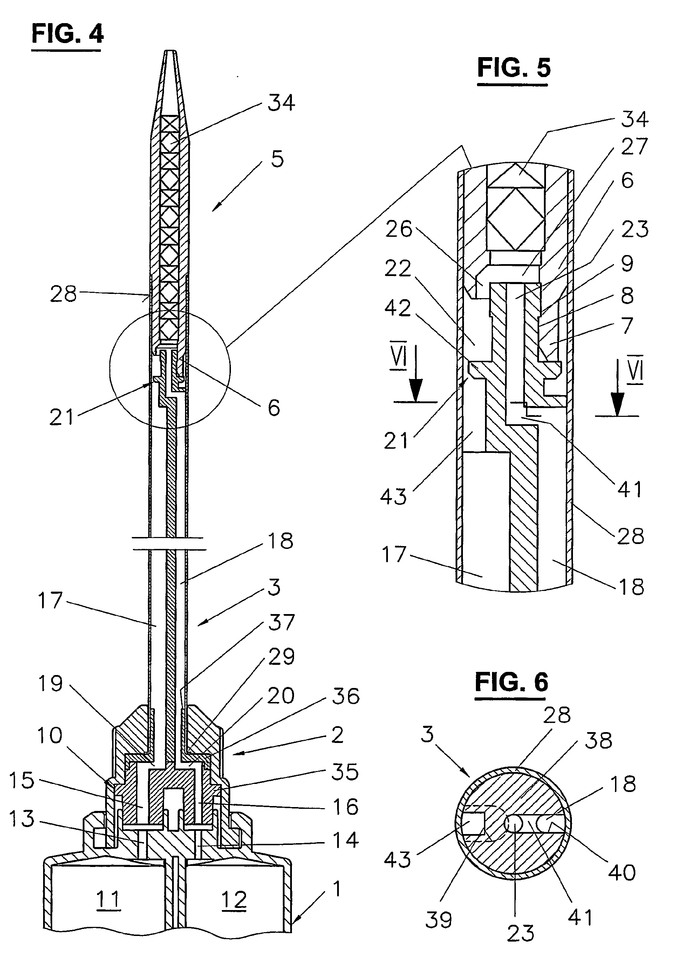

Die Kanäle 17 und 18 liegen hier weniger weit auseinander als die beiden Auslässe 13 und 14 der Kartusche, wobei die Kanäle 15 und 16 des Eingangs-Adapters derart geformt sind, dass seine Auslässe 19 und 20 mit den Kanälen 17 und 18 des Transferteils übereinstimmen. Am kartuschenseitigen Ende weist das Mantelrohr 28 eine Bördelung 29 auf, um von der Schulter des Überwurfrings erfasst zu werden, wie dies aus den Figuren 1 oder 4 hervorgeht.

Am anderen Ende des Transferteils befindet sich ein Ausgangs-Adapter 21 mit zum Zentralkanal 23 konzentrisch angeordneten Kanälen 22, deren Auslässe entweder mit den Einlässen 24 und 25 der Ausgleichvorrichtung oder mit den Einlässen 26 und 27 des Mischers kommunizieren. Das Mantelrohr 28 ragt über den Ausgangs-Adapter hinaus, so dass entweder der entsprechend geformte Eingang der Ausgleichvorrichtung oder der Mischereingang in dieses Rohrteil hineingeschoben werden kann, wodurch zusätzlich eine Abstützung des Mischers erzielt wird.

Wie aus Figur 6 hervorgeht, ist der aussen liegenden Kanal 18 über einen Querkanal 41 mit dem etwa in der Mitte angeordnete Kanal 23 verbunden. Der in der Zeichnung links liegende Kanal 17 mündet in einen Rechteckkanal 43, der bis zu einer umlaufende Schulter 42 im Ausgangsadapter verläuft, wobei die Schulter einen kleineren Durchmesser aufweist als das Mantelrohr, so dass das Material diese umfliessen kann. Oberhalb der umlaufenden Schulter 42 sind die Kanäle 22 konzentrisch um Kanal 23 angeordnet. Die Schulter 42 dient auch als Auflage für den Mischer.

Die Ausgleichvorrichtung 4 weist ein Gehäuse 30 sowie zwei Kammern 31 und 32 auf und ist mit einem porösen Filter 33 abgeschlossen, das im Wesentlichen nur luftdurchlässig ist, jedoch das Material zurückzuhalten vermag. Es ist auch möglich, statt eines porösen Filters einen Deckel mit einer oder mehreren Kapillaröffnung(en) vorzusehen. Vorzugsweise ist das Gehäuse der Ausgleichvorrichtung aus einem durchsichtigen Material gefertigt, so dass visuell festgestellt werden kann, ob der Füllstand der beiden Vorratszylinder angeglichen und das Transferteil vollständig mit den Komponenten gefüllt ist. Wichtig ist hier, dass die Komponenten gleichzeitig in den Mischer gelangen und dadurch von Anfang des Austragens in der Bauchhöhle eine klebfähige Mischung erzielt wird.

In den Figuren 1 und 2 ist die Ausgleichvorrichtung auf dem Transferteil aufgesetzt und es ist ersichtlich, dass die Kanäle 22 und 23 des Ausgang-Adapters mit den Einlässen 24, 25 der Ausgleichvorrichtung kommunizieren. Nach dem Abziehen der Ausgleichvorrichtung liegen die Füllniveaus der beiden Komponenten auf unterschiedlichen Höhen, so dass gewährleistet ist, dass zu diesem Zeitpunkt keine Reaktion stattfinden kann.

In den Figuren 4 und 5 ist dieselbe Kartusche und derselbe Überwurfring sowie dasselbe Transferteil mit Ausgangs-Adapter 21 dargestellt, wobei auf dem Transferteil ein Mischer 5 aufgesteckt ist. Der Eingang des Mischers weist ein Anschlussteil 6 auf, an dem sich federnde Zungen 7 mit einem Absatz 8 befinden, der eine entsprechende Stufe 9 im Ausgangs-Adapter hintergreift und nach dem Hineinstecken in das Rohr des Transferteils sein Herausziehen verhindert. Der Mischer weist die an sich bekannten Mischelemente 34 auf.

Es ist aus den Figuren 4 und 5 ersichtlich, dass der mittlere, zentral angeordnete Kanal 23 des Ausgangs-Adapters konzentrisch von anderen Kanälen 22, bzw. den Zungen des Mischereingangs umgeben ist und mit den Mischer-Einlässen 26 und 27 kommunizieren.

Der Arbeitsablauf kann wie folgt beschrieben werden: Nach Entfernen der Verschlusskappe von der gefüllten Kartusche wird das vormontierte Transferteil mitsamt dem Eingangs-und Ausgangs-Adapter mittels dem Überwurfring an der Kartusche befestigt. Daraufhin wird die Ausgleichvorrichtung bis zum Anschlag auf das Transferteil eingeschoben und die ganze Anordnung senkrecht mit der Ausgleichvorrichtung nach oben gerichtet, um ein Entweichen der Luft zu ermöglichen.

Anschliessend wird Material ausgetragen, bis beide Komponenten in der Ausgleichvorrichtung sichtbar sind. Dadurch ist sichergestellt, dass beide Komponenten von Anbeginn des Austragens gleichzeitig in den Mischer gelangen, um von Anfang an eine klebfähige Mischung zu erhalten. Ausserdem wird mit diesen Massnahmen erreicht, dass damit auch die beiden Vorratszylinder der Kartusche sowie das Transferteil luftfrei sind.

Dann wird die Ausgleichvorrichtung abgezogen und entsorgt und das Anschlussteil des Mischers in das Tranferteil-Rohr gesteckt und bis zum Einrasten eingeschoben, womit der Mischer für die Verarbeitung vorbereitet ist.

Infolge der unterschiedlichen Kanalgestaltung im Auslassteil, bzw. im Einlassteil der Ausgleichvorrichtung oder im aufgesteckten Mischer wird vermieden, dass die beiden Komponenten miteinander in Kontakt kommen können.

Im obigen Ausführungsbeispiel wurde eine Kartusche mit nebeneinanderliegenden Auslässen und nebeneinanderliegenden Einlässen des Eingangs-Adapters sowie nebeneinanderliegende Kanäle des Transferteils beschrieben, die in konzentrische Kanäle des Ausgangs-Adapters münden und in die konzentrischen Einlässe des Mischers oder Einlässe der Ausgleichvorrichtung übergehen. Es ist jedoch auch möglich, dieses System Kartusche-Transferteil-Ausgleichvorrichtung oder Mischer bei einer Anordnung vorzusehen, bei denen die beiden Komponenten überall oder teilweise konzentrisch zueinander angeordnet sind, wobei die Adapter entsprechend ausgebildet sind.

Ausserdem ist es möglich, das Austraganordnung auf die Verwendung von drei Komponenten zu erweitern.

Bei der Anwendung der erfindungsgemässen Anordnung in der Endoskopie ist es selbstverständlich, dass die Dimensionen und die Ausgestaltung des Transferteils und des Mischers dem Innendurchmesser der Endoskoprohre angepasst sein müssen, wobei der Mischer nirgends eine grösseren Aussendurchmesser haben darf als der Aussendurchmesser des Rohres des Transferteils, jedoch gegen den Auslass hin konisch verjüngt sein kann. Bei anderen Anwendungen ist es selbstredend möglich, andere Dimensionen zu vorzusehen.

## Patentansprüche

1. Austraganordnung für zwei Komponenten mit einer Austragkartusche oder einem Austraggerät und einem Mischer, wobei zwischen der Austragkartusche (1) oder Austraggerät und dem Mischer (5) ein Transferteil (3) angeordnet ist, das sowohl mit der Kartusche oder Gerät als auch mit dem Mischer verbindbar ist und mindestens zwei Durchgänge enthält,
**dadurch gekennzeichnet, dass** das Transferteil (3) eine zylinderförmige Stange (38) aufweist, in deren Umfang zwei Längsnuten (39, 40) angeordnet sind und die Stange von einem Mantelrohr (28) umschlossen ist, um zwei Kanäle (17, 18) zu bilden, derart dicht, dass kein Material von einem Kanal zum anderen gelangen kann und dass die Austraganordnung eine Ausgleichvorrichtung (4) aufweist, die mit dem Transferteil (3) verbindbar ist, um den Füllstand der Komponenten auszugleichen und die Austraganordnung zu entlüften, und danach wieder abgenommen werden kann, so dass der Transferteil (3) mit dem mischer (5) verbindbar ist.

2. Austraganordnung nach Anspruch 1, **dadurch gekennzeichnet, dass** beidseitig des Transferteils ein Adapter (10, 21) angeordnet ist.

3. Austraganordnung nach Anspruch 1 oder 2, **dadurch gekennzeichnet, dass** der Adapter (10; 21) an einem Ende Einlässe (15, 16) und am anderen Ende Auslässe (19, 20) aufweist, die mit den Auslässen (13, 14) der Kartusche, bzw. Einlässen (24, 25) der Ausgleichvorrichtung oder (26, 27) des Mischers einerseits und den Kanälen (17, 18) des Transferteils kommunizieren.

4. Austraganordnung nach einem der Ansprüche 1 bis 3, **dadurch gekennzeichnet, dass** am Mischereinlass ein Mischeranschlussteil (6) angeordnet ist, das in das überstehende Rohr (28) des Transferteils steckbar und mit dem Ausgangs-Adapter (21) am Transferteil (3) verbindbar ist und federnde Zungen (7) aufweist, die mit einem Absatz (8) versehen sind, der mit einem entsprechenden Absatz im Ausgangadapter (21) kooperiert.

5. Austraganordnung nach Anspruch 4, **dadurch gekennzeichnet, dass** der Ausgangs-Adapter (21) zwei konzentrisch angeordnete Auslässe (22, 23) und der Mischereinlass zwei konzentrisch angeordnete Einlässe (24, 25) aufweisen.

6. Austraganordnung nach einem der Ansprüche 1 bis 5, **dadurch gekennzeichnet, dass** die Ausgleichvorrichtung ein Gehäuse (30) mit zwei getrennten Kammern (31, 32) aufweist, die aus einem durchsichtigen Material gefertigt sind, wobei das Gehäuse durch ein poröses Filter (33) oder eine oder mehrere Kapillaröffnung(en) abgeschlossen ist.

7. Austraganordnung nach einem der Ansprüche 1 bis 6, **dadurch gekennzeichnet, dass** die Kartuschen-Auslässe und/oder die Transferteil-Einlässe und/oder -Kanäle und/oder -Auslässe nebeneinander oder konzentrisch zueinander angeordnet sind.

8. Austraganordnung nach einem der Ansprüche 1 bis 7, **dadurch gekennzeichnet, dass** die Mischer- und/oder Ausgleichvorrichtung-Einlässe nebeneinander angeordnet sind.

## Claims

1. Dispensing assembly for two components, including a dispensing cartridge or a dispensing appliance and a mixer, a transfer portion (3) being interposed between the dispensing cartridge (1) or appliance and the mixer (5) which is connectable both to the cartridge or appliance and to the mixer and comprises at least two ducts, **characterised in that** the transfer portion (3) comprises a cylindrical bar (38) at the periphery of which two longitudinal grooves (39, 40) are arranged, and the bar is enclosed in a jacket (28) in order to form two channels (17, 18) that are tight such that no material may pass from one channel to the other, and **in that** the dispensing assembly comprises a compensating device (4) that is connectable to the transfer portion (3) in order to align the component levels and to deareate the dispensing assembly, and detachable subsequently so that the transfer portion (3) is connectable to the mixer (5).

2. Dispensing assembly according to claim 1, **characterised in that** an adapter (10, 21) is provided on each side of the transfer portion.

3. Dispensing assembly according to claim 1 or 2, **characterised in that** the adapter (10; 21) comprises inlets (15, 16) at one end and outlets (19, 20) at the other end, which communicate with the outlets (13, 14) of the cartridge resp. with the inlets (24, 25) of the compensating device or (26, 27) of the mixer, on one hand, and with the channels (17, 18) of the transfer portion.

4. Dispensing assembly according to one of claims 1 to 3, **characterised in that** the mixer inlet is provided with a mixer connecting portion (6) which is insertable into the projecting tube (28) of the transfer portion and connectable to the exit adapter (21) on the transfer portion (3), and which comprises elastic tongues (7) that are provided with a shoulder (8) cooperating with a corresponding shoulder in the exit adapter (21).

5. Dispensing assembly according to claim 4, **characterised in that** the exit adapter (21) comprises two concentrically disposed outlets (22, 23) and the mixer inlet comprises two concentrically disposed inlets (24, 25).

6. Dispensing assembly according to one of claims 1 to 5, **characterised in that** the compensating device comprises an enclosure (30) having two separate chambers (31, 32) that are made of a transparent material, the enclosure being terminated by a porous filter (33) or one or a plurality of capillary opening(s).

7. Dispensing assembly according to one of claims 1 to 6, **characterised in that** the cartridge outlets and/or the transfer portion inlets and/or channels and/or outlets are arranged side by side or concentrically.

8. Dispensing assembly according to one of claims 1 to 7, **characterised in that** the inlets of the mixer and/or the compensating device are arranged side by side.

## Revendications

1. Agencement de distribution pour deux composants avec une cartouche de distribution ou un applicateur et un mélangeur, entre la cartouche de distribution (1) ou l'applicateur et le mélangeur (5) étant interposé un élément de transfert (3) qui peut être relié à la fois à la cartouche ou l'applicateur et au mélangeur et comprend au moins deux passages, **caractérisé en ce que** l'élément de transfert (3) comprend une tige (38) cylindrique pourvue de deux rainures longitudinales (39, 40) à sa circonférence, et que la tige est entourée d'un tube d'enveloppe (28) afin de former deux canaux (17, 18) étanches de telle manière qu'aucune matière ne peut passer d'un canal à l'autre, et **en ce que** l'agencement de distribution comporte un dispositif d'égalisation (4) pouvant être relié à l'élément de transfert (3) pour égaliser le niveau des composants et désaérer l'agencement de distribution, et enlevé par la suite pour relier l'élément de transfert (3) au mélangeur (5).

2. Agencement de distribution selon la revendication 1, **caractérisé en ce qu'**un adaptateur (10, 21) est agencé de chaque côté de l'élément de transfert.

3. Agencement de distribution selon la revendication 1 ou 2, **caractérisé en ce que** l'adaptateur (10; 21) présente à une extrémité des entrées (15, 16) et à l'autre extrémité des sorties (19, 20) qui communiquent d'une part avec les sorties (13, 14) de la cartouche et les entrées (24, 25) du dispositif d'égalisation ou (26, 27) du mélangeur, respectivement, et avec les canaux (17, 18) de l'élément de transfert.

4. Agencement de distribution selon l'une des revendications 1 à 3, **caractérisé en ce que** l'admission du mélangeur est pourvue d'un raccord de mélangeur (6) qui est enfichable dans le tube (28) en saillie de l'élément de transfert et connectable à l'adaptateur de sortie (21) sur l'élément de transfert (3) et qui présente des languettes (7) élastiques munies d'un talon (8) coopérant avec un talon correspondant dans l'adaptateur de sortie (21).

5. Agencement de distribution selon la revendication 4, **caractérisé en ce** l'adaptateur de sortie (21) présente deux sorties (22, 23) agencés concentriquement et l'admission du mélangeur deux entrées (24, 25) agencés concentriquement.

6. Agencement de distribution selon l'une des revendications 1 à 5, **caractérisé en ce** le dispositif d'égalisation comprend un boîtier (30) ayant deux chambres séparées (31, 32) en une matière transparente, le boîtier étant fermé par un filtre (33) poreux ou une ou plusieurs ouvertures capillaires.

7. Agencement de distribution selon l'une des revendications 1 à 6, **caractérisé en ce que** les sorties de la cartouche et/ou les entrées et/ou canaux et/ou sorties de l'élément de transfert sont agencés côte à côte ou concentriquement.

8. Agencement de distribution selon l'une des revendications 1 à 7, **caractérisé en ce que** les entrées du mélangeur et/ou du dispositif d'égalisation sont agencés côté à côte.
